# FASCICULE DE BREVET EUROPEEN

(11) **EP 0 731 676 B1**
(45) Date de publication et mention de la délivrance du brevet: **21.05.1997**
(21) Numéro de dépôt: 95902164.3
(22) Date de dépôt: 22.11.1994
(51) Int. Cl.: A61F 2/38

(54) **PROTHESE TOTALE DE GENOU ET ENSEMBLE PROTHETIQUE MODULABLE DE GENOU CORRESPONDANT**
GANZKNIEPROTHESE SOWIE ENTSPRECHENDER MODULARER KNIEPROTHESENBAUSATZ
TOTAL KNEE PROSTHESIS AND ASSOCIATED MODULAR KNEE PROSTHESIS ASSEMBLY

(30) Priorité: 22.11.1993 FR 9313952
(43) Date de publication de la demande: 18.09.1996
(73) Titulaire: LANDANGER-LANDOS, Société Anonyme, F-52003 Chaumont (FR)
(72) Inventeur: BREMAND, Jean-Jacques, F-73000 Chambéry (FR); CAILLE, Jean-Pierre, F-26000 Valence (FR); CALTRAN, Maurice, Clesses (FR); CHAMBAUD, Denis, F-42153 Riorges (FR); CROUZET, Marc, F-69250 Ouris-au-Mont-d'Or (FR); IMBERT, Jean-Claude, F-42680 L'Etrat (FR); LANDANGER, Patrick, F-52000 Chaumont (FR); LECLERC-CHALVET, François, Buxy (FR); LERAT, Jean-Luc, F-69437 Lyon Cédex 03 (FR); MARCEAUX, Pascal, F-52000 Chaumont (FR); MOYEN, Bernard, F-69437 Lyon Cédex 03 (FR); PAILLOT, Jean-Michel, F-73100 Aix-les-Bains (FR); TREBAL, Jean-Pierre, F-75011 Paris (FR)
(74) Mandataire: Martin, Jean-Paul
(86) Numéro de dépôt international: FR9401362
(87) Numéro de publication internationale: WO9514446

(56) Documents cités:
- EP-A- 0 336 774
- EP-A- 0 510 299
- FR-A- 2 668 703

## Description

La présente invention a pour objet une prothèse totale de genou, comportant un implant fémoral comprenant une partie antéro-postérieure, une trochlée, un condyle externe et un condyle interne, et un plateau tibial coopérant avec l'implant fémoral. Complémentairement l'invention concerne également un ensemble prothétique incorporant cette prothèse totale.

L'implant fémoral constitue un carter anatomique partant de la reproduction de la partie distale du fémur et posée à l'extrémité de celle-ci après les resections osseuses nécessaires.

Comme on le sait, les implants fémoraux sont généralement constitués d'éléments métalliques massifs réalisés de façon à enserrer fermement la partie distale du fémur. Les deux condyles assurent la congruence avec le plateau tibial et peuvent être associés à un troisième élément soudé à la partie antéro-postérieure entre les deux condyles. (FR-2668703) Ce troisième élément a pour fonction d'assurer une postéro-stabilisation du genou en cas de défaillance ou de destruction du ligament croisé postérieur, en coopération avec le plateau tibial, lorsque le genou subit une flexion telle qu'elle peut provoquer un échappement de l'implant fémoral par rapport au plateau tibial et ainsi une subluxation du genou.

Pour mettre en oeuvre de telles prothèses de genou, le chirurgien doit donc avoir à sa disposition deux jeux de plusieurs tailles, parmi lesquelles il choisit celle qui correspond le mieux aux dimensions osseuses de son patient, à savoir un premier jeu sans élément central postéro-stabilisateur au cas où le ligament croisé postérieur est encore en bon état, et un second jeu complet de prothèses avec élément central postéro stabilisateur au cas où le ligament croisé postérieur est défaillant ou doit être supprimé. Le choix du modèle et de la taille adaptée peut être fait en per-opératoire par le chirurgien. En pratique, chaque jeu est généralement constitué de six prothèses, et parfois plus : cela rend l'ensemble prothétique extrêmement onéreux et d'une gestion relativement difficile pour le chirurgien. Le coût de ces jeux de prothèses est d'autant plus élevé qu'elles sont généralement réalisées en un alliage de titane ou de chrome-cobalt, qui sont des matériaux très onéreux.

Il faut également observer qu'à chaque jeu d'implants fémoraux doit nécessairement correspondre un plateau tibial profilé de manière distincte, ce qui accroît la lourdeur de l'ensemble.

L'invention a donc pour but de réaliser une prothèse totale de genou ainsi qu'un ensemble prothétique permettant de réduire notablement le nombre total de pièces nécessaires, donc le coût de fabrication de l'ensemble, tout en offrant un maximum de souplesse au chirurgien dans le choix des prothèses appropriées à l'état fonctionnel du ligament croisé postérieur. Cela peut être, soit avant l'opération chirurgicale par l'examen des radiographies du patient, ou pendant cette opération en fonction de ce qu'il constate visuellement sur l'état réel du genou.

Conformément à l'invention, l'implant fémoral est pourvu de moyens amovibles de butée de postéro-stabilisation, positionnés entre les deux condyles, et coopérant avec une entaille correspondante ménagée dans la surface du plateau tibial.

Suivant un mode de réalisation de l'invention, ces moyens de butée sont constitués par un troisième condyle, solidaire d'une plaque équipée de moyens de fixation amovible à la partie antéro-postérieure, par exemple au moins un plot et de préférence deux plots d'ancrage fémoral traversant la plaque et dont une extrémité est vissée dans la partie antéro-postérieure.

Suivant une autre caractéristique de l'invention, la plaque portant le troisième condyle est placée dans un logement réalisé dans la partie antéro-postérieure et dont la profondeur est pratiquement égale à l'épaisseur de la plaque, la surface de cette dernière affleurant par conséquent sensiblement la surface de la partie antéro-postérieure.

L'ensemble prothétique également prévu par l'invention comprend une seconde plaque dans le bord antérieur de laquelle est réalisée une échancrure incurvée dont le contour épouse celui des bords en vis-à-vis des condyles externe et interne, au moins un trou de préférence deux étant formés dans cette plaque pour recevoir un ou deux plots correspondants dont les extrémités viennent se visser dans des trous du fond du logement, pour fixer la plaque de manière amovible à la partie antéro-postérieure.

Ainsi, en fonction de l'état du ligament croisé postérieur, le chirurgien peut choisir entre deux solutions, soit utiliser la plaque équipée du condyle central, soit utiliser la plaque dépourvue de condyle central. Dans le second cas l'implant fémoral se présente extérieurement de manière absolument similaire à un implant fémoral classique. On comprend que de ce fait un seul jeu d'implants fémoraux au lieu de deux jeux suffit pour permettre au chirurgien d'effectuer le choix de la prothèse adaptée à l'état du ligament croisé postérieur, ainsi qu'à l'anatomie osseuse du patient.

Avantageusement les faces internes du carter fémoral peuvent être revêtues d'une couche d'hydroxyapatite de calcium ainsi que les faces externes de la plaque équipée du condyle central et de la plaque dépourvue de cet élément. Le dépôt de cette couche est alors effectué à l'aide d'une torche à plasma permettant de projeter la couche d'hydroxyapatite sur la surface métallique du carter fémoral préalablement préparée pour obtenir une adhérence suffisante sur le métal.

On sait en effet qu'un processus d'adsorption se produit entre le tissu osseux et l'hydroxyapatite assurant ainsi un ancrage complémentaire de la prothèse sur la partie distale du fémur.

D'autres particularités et avantages de l'invention apparaîtront au cours de la description qui va suivre, faite en référence aux dessins annexés qui en illustrent une forme de réalisation à titre d'exemple non limitatif.

La figure 1 est une vue en perspective éclatée d'une forme de réalisation de la prothèse totale de genou conforme à l'invention.

La figure 2 est une vue en perspective éclatée sous un autre angle de l'implant fémoral de la figure 1, montrant notamment la plaque équipée du condyle central et son logement dans la partie antéro-postérieure.

La figure 3 est une vue en perspective de l'implant fémoral de la figure 2 du côté des surfaces d'appui des condyles sur le plateau tibial.

La figure 4 est une vue en perspective du plateau tibial coopérant avec l'implant fémoral des figures 2 et 3.

La figure 5 est une vue en coupe partielle de la plaque et de la partie antéro-postérieure de l'implant fémoral des figures 1 à 3.

La figure 6 est une vue de dessus d'une plaque dépourvue de butée postéro-stabilisatrice, pouvant remplacer la plaque visible aux figures 1 et 2.

La figure 7 est une vue en élévation longitudinale de la plaque de la figure 6.

La figure 8 et une vue en élévation latérale et coupe partielle d'une seconde forme de réalisation de l'implant fémoral.

La prothèse totale de genou représentée aux dessins comporte un implant fémoral 1 et un plateau tibial 2 de support de cet implant fémoral, lequel doit être fixé à la partie distale d'un fémur, tandis que le plateau tibial 2 est ancré sur l'extrémité en vis à vis d'un tibia.

L'implant fémoral 1 comprend une partie antéro-postérieure 3, une trochlée 4, un condyle externe 5 et un condyle interne 6, dont les surfaces sont profilées anatomiquement pour pouvoir assurer la congruence avec des surfaces anatomiques complémentaires 7, 8 du plateau tibial 2.

L'implant fémoral 1 est pourvu de moyens amovibles de butée de postéro-stabilisation, positionnés entre les deux condyles 5 et 6 et adaptés pour coopérer avec une entaille profilée correspondante 9 ménagée dans la surface du plateau tibial 2 entre les surfaces anatomiques 7 et 8.

Dans le mode de réalisation représenté, ces moyens amovibles de butée sont constitués par un troisième condyle central 11 solidaire d'une plaque 12 dimensionnée et profilée pour pouvoir être placée dans un logement 13 réalisé dans la partie antéro-postérieure 3, et dont la profondeur est égale à l'épaisseur de la plaque 12.

La plaque 12 est équipée de moyens de fixation amovibles à la partie 3, par exemple au moins un plot 14 d'ancrage fémoral, et de préférence deux plots 14 comme représenté aux dessins. Ces plots 14 comportent chacun une extrémité filetée 14a et une extrémité opposée pourvue de méplats 10 permettant leur vissage par une clé non représentée. Ils peuvent traverser des trous 15 formés dans la plaque 12, de chaque côté du condyle central 11, de telle sorte que leurs extrémités filetées 14a puissent être vissées dans des trous taraudés complémentaires 16 usinés dans le fond du logement 13, de chaque côté d'une échancrure 17 séparant les deux condyles 5 et 6. Les parties filetées 14a sont limitées, du côté opposé à leurs extrémités libres, par un ressaut tronconique 18 formant épaulement d'appui sur la surface de la plaque 12 autour des trous 15 lorsque les extrémités filetées 14a sont vissées dans les trous 16, afin d'assurer un serrage solide de la plaque 12 sur la partie antéro-postérieur 3.

Les plots 14 ont une longueur suffisante pour pouvoir assurer un ancrage osseux convenable dans le fémur, dont à cet endroit la structure est en effet spongieuse.

Avantageusement la plaque 12 comporte un rebord périphérique 19 raccordé au condyle 11 par une surépaisseur centrale 20, et formant un bourrelet saillant de la surface de la plaque 12 et de celle de la partie 3. Le bord antérieur 13a du logement 13 est profilé en queue d'aronde (ou en variante par tout autre profil similaire), adapté pour recevoir un bord antérieur 12a de la plaque 12, profilé de manière conjuguée pour pouvoir s'appliquer sur le profil en surplomb du bord 13a (Figure 5).

L'entaille 9 du plateau tibial 2 et le condyle central 11 sont conformés de manière à empêcher une subluxation en cas de flexion du genou, le profil de l'entaille 9 étant en effet tel qu'il s'oppose à tout échappement intempestif du condyle 11 et donc de l'implant fémoral 1 par rapport au plateau tibial 2. En outre, la surface du plateau 2 dans son ensemble est complémentaire des surfaces anatomiques des condyles 5 et 6.

Le logement 13 peut recevoir, au lieu de la plaque 12, une autre plaque 21 dépourvue de condyle, et dans le bord antérieur de laquelle est réalisée une échancrure incurvée 22 dont le contour épouse celui de l'échancrure 17, elle-même délimitée par les bords en vis-à-vis des condyles 5 et 6. Deux trous taraudés 23 sont de plus agencés dans la plaque 21, de part et d'autre de l'échancrure 22, et peuvent recevoir les parties terminales filetées 14a de deux plots correspondants 14 de fixation de la plaque 21 à la partie antéro-postérieure 3.

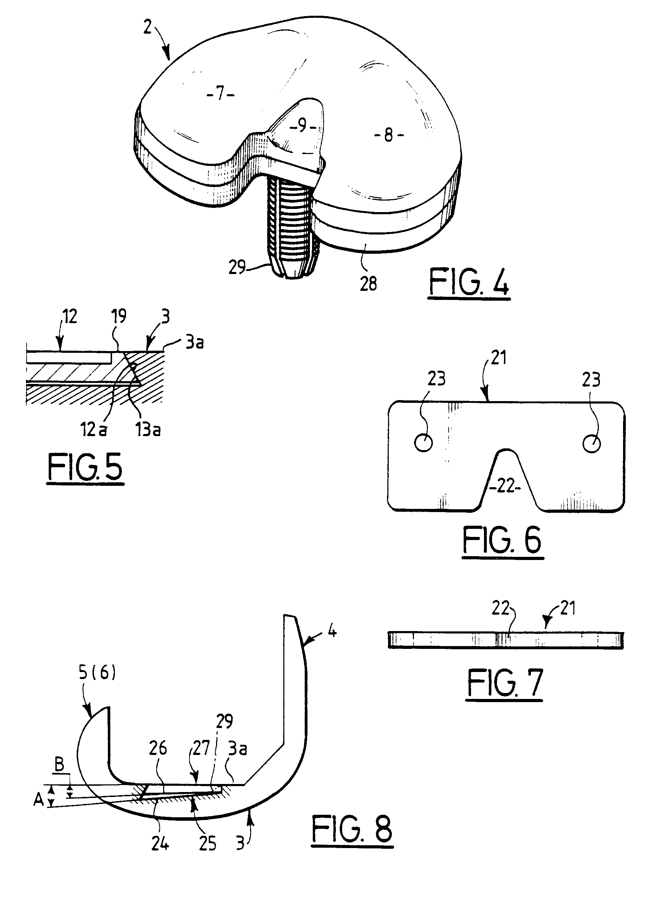

On a représenté à la figure 8 un second mode de réalisation de l'implant 1, dans lequel le fond 24 du logement 25 est incliné d'un angle A vers les condyles 5, 6 et 11 par rapport à la surface 3a de la partie antéro-postérieure 3. Complémentairement la face 26 de la plaque 27, destinée à venir en appui sur le fond 24 du logement 25, est également inclinée, lorsque la plaque est mise en place dans le logement 25, sur la surface 3a de la partie 3 d'un angle B légèrement inférieur à celui A du fond 24.

Ces angles sont faibles, et à titre d'exemple non limitatif, A peut être de 4 degrés tandis que B est de 3 degrés.

En positionnant les plots 14 dans la moitié postérieure de la plaque 27 on réalise alors pendant leur vissage un effet de bascule sur la plaque 27, qui l'applique et la maintient fermement en appui sur d'une part la plus grande partie du fond 24, et d'autre part sur la demi-queue d'aronde 13a par son bord antérieur 12a. Un serrage solide des plots 14 assure le maintien dans le temps de ce contact, ce qui évite tout risque de micro-vibrations de la plaque sur la partie 3. Les micro-vibrations sont en effet à l'origine des problèmes de corrosion, qu'il est impératif d'éviter dans l'intérêt du patient.

Bien entendu, la plaque 27 peut être avec ou sans condyle central 11, donc dans le second cas être similaire à la plaque 21.

L'ensemble de l'implant fémoral 1, des plaques 12 et 21 et du plateau tibial 2 forme un ensemble prothétique modulable de genou, dont la mise en oeuvre par le chirurgien se fait de la manière suivante.

Si celui-ci estime que le ligament croisé postérieur est dans un état tel qu'il ne peut plus remplir sa fonction normale (ramener le genou vers l'avant pour empêcher une subluxation), ou si ce ligament croisé postérieur est complètement absent, il choisit, si nécessaire en phase per-opératoire, un carter fémoral ou implant 1 et une plaque 12 adaptés à la taille du patient. La plaque 12 est placée dans son logement 13, en emboîtant les profils complémentaires 12a et 13a l'un dans l'autre, de manière à bien positionner la plaque 12. Puis le chirurgien procède à la fixation de cette dernière à la partie antéro-postérieure 3 en vissant les parties filetées 14a des plots 14 dans les trous 15 et 16. Ensuite, les plots 14 sont introduits dans le fémur pour assurer l'ancrage osseux de l'implant fémoral 1. Le plateau tibial 2 est de son côté fixé à l'extrémité supérieure du tibia par des moyens connus en soi tels qu'une embase 28 et un axe 29 (figure 4).

Si par contre l'état du ligament croisé postérieur rend inutile la mise en oeuvre de la plaque 12 et des moyens de butée 11 et 9, le chirurgien a la faculté de choisir la plaque 21, qui vient remplir le logement 13. La fixation est assurée de la même manière que précédemment par un ou deux plots 14, au travers des trous 23 et 16.

En plus des avantages précédemment indiqués, la prothèse totale du genou et l'ensemble prothétique qui vient d'être décrit présente les suivants.
- Le bourrelet périphérique saillant 19 renforce la résistance mécanique de la plaque 12 et peut être impacté dans l'os, sans sacrifice osseux supplémentaire.
- Les profils complémentaires en queue d'aronde 12a, 13a sur le bord antérieur de la plaque 12 et du logement 13 encaissent les efforts dirigés vers l'avant, ce qui soulage d'autant le condyle central 11. De plus, ces profils complémentaires évitent des micro-mouvements de la plaque 12, qui pourraient éventuellement provoquer un déscellement osseux, surtout s'ils sont combinés avec les inclinaisons A et B du fond 24 et de la face d'appui 26 de la plaque.
- Le troisième condyle 11 est dimensionné pour ne pas gêner les mouvements des deux autres, en évitant de les faire osciller latéralement, et pour pouvoir jouer son rôle de butée en fin de basculement de l'implant fémoral 1 sur le plateau tibial 2.
- Le plateau 2 peut coopérer, soit avec un carter fémoral classique, soit avec un carter fémoral à post-stabilisateur, c'est à dire l'implant 1 pourvu de la plaque 12 et du condyle central 11. Il s'agit là d'un avantage important par rapport à l'état de la technique antérieure connue, ce rôle polyvalent du plateau tibial 2 étant rendu possible par le fait que les surfaces 7 et 8 sont complémentaires des surfaces des condyles 5 et 6 qui sont réalisées de manière anatomique, conformément à une particularité de l'invention. En effet, dans les ensembles prothétiques antérieures connus, les surfaces du plateau tibial et de l'implant fémoral ne sont pas anatomiques et donc conjuguées l'une de l'autre. De ce fait, il est nécessaire de disposer d'un plateau tibial adapté à un implant fémoral sans poststabilisateur, et d'un autre plateau tibial distinct adapté à un implant fémoral avec poststabilisateur soudé à la partie antéro-postérieure.

Suivant une caractéristique complémentaire avantageuse, les surfaces des deux plaques 12 et 21 ainsi que de la partie antéro-postérieure 3 peuvent être revêtues d'une couche d'hydroxyapatite de calcium, réalisée à l'aide d'une torche à plasma, ce matériau favorisant la repousse osseuse et donc l'ostéointégration de la prothèse.

Bien entendu, le revêtement d'hydroxyapatite de calcium est exclu dans le logement 13 destiné à recevoir l'une ou l'autre des plaques 12 et 21 destinée à être en contact avec le fond du logement.

Il est également possible, dans un carter fémoral de l'invention dont il n'a pas été prévu de réaliser une couche d'hydroxyapatite sur toute sa surface intérieure, de choisir une plaque 12 ou 21 dont la surface externe, hormis le condyle 11 pour la plaque 12, a été revêtue d'hydroxyapatite de calcium. Cette solution peut constituer pour le chirurgien une option supplémentaire permettant d'accroître aisément la qualité de la fixation du carter dans la partie distale du fémur.

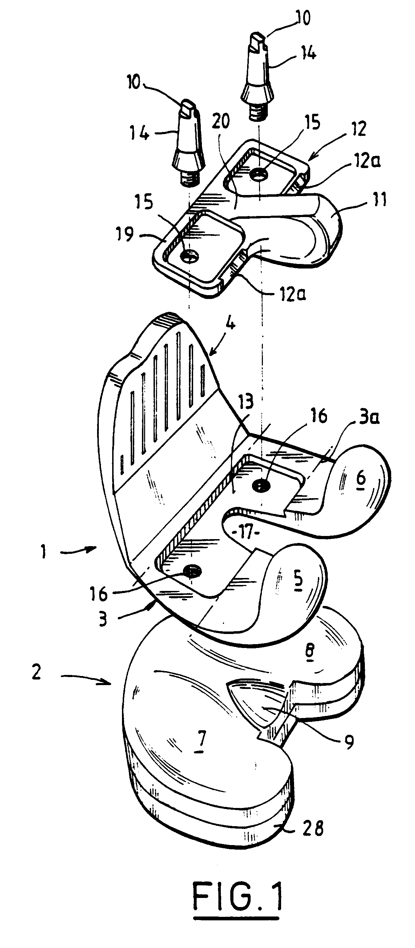

A la figure 1, on voit en trait mixte une variante d'exécution selon laquelle le logement 13 s'étend sur toute la largeur de la partie antéro-postérieure 3. Cet agencement permet d'usiner plus facilement le logement de la plaque 12, et rend indispensables les plots de fixation 14 - alors qu'ils ne le sont pas dans la réalisation de la Fig.1 grâce au profil en queue d'aronde (ou similaire) du bord 13a du logement 13.

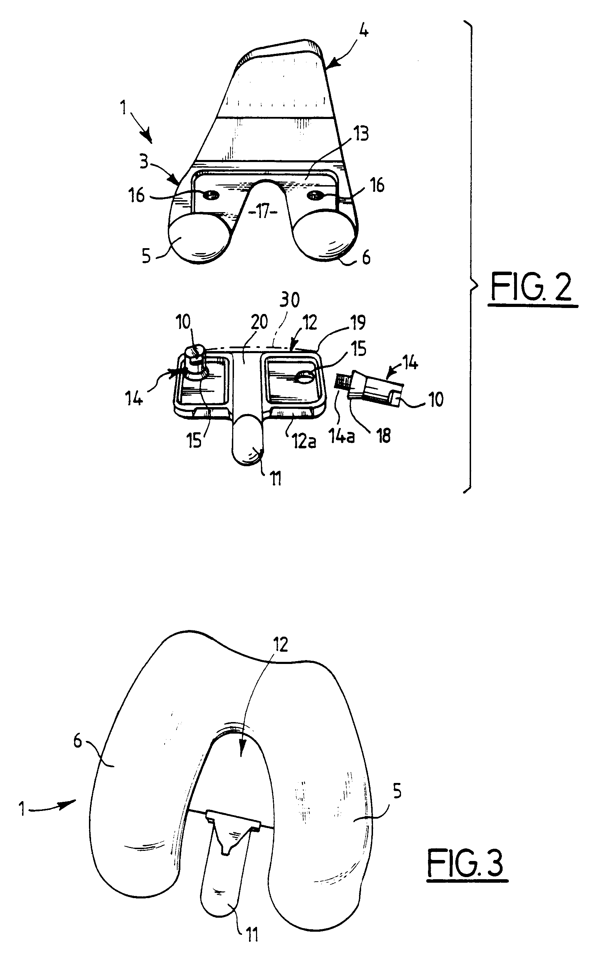

Selon une autre variante possible, illustrée à la Fig.2, la plaque amovible 12 présente un bord arrière 30 (en trait mixte), légèrement convexe. De ce fait, lorsque la plaque 12 ainsi modifiée est insérée dans son logement 13, la pression exercée au centre du bord 30 est suffisante pour provoquer, grâce à la légère élasticité du matériau, une légère extension latérale qui accroît la solidarisation latérale de la plaque avec la partie antéro-postérieure 3. Eventuellement, cet accroissement de pression et de la force de solidarisation plaque 12 - partie 3 peut rendre superflus les plots d'ancrage 14 qui sont prévus pour assurer une sécurité supplémentaire. En effet, l'insertion de la plaque 12 dans son logement 13 crée une répartition des forces sur le pourtour de ce dernier.

Enfin, il est possible de ménager un chanfrein 29 sur le côté arrière du logement 25 (Fig.8). Ce chanfrein facilite l'autopositionnement de la plaque 27.

L'invention est susceptible de diverses autres variantes d'exécution. Ainsi il est évident que les profils complémentaires en queue d'aronde 12a, 13a peuvent être remplacés par tout autre profil équivalent. De même, les plaques 12 et 21 peuvent avoir leurs bords antérieurs et postérieurs profilés de manière complémentaire de deux bords antérieur et postérieur correspondant du logement 13, en queue d'aronde ou selon tout autre profil équivalent. Les plaques 12 et 21 peuvent également être solidarisées avec la partie antéro-postérieure par une colle biocompatible remplaçant les plots 14, ou par tout autre moyen équivalent.

## Revendications

1. Prothèse totale de genou, comportant un implant fémoral (1) comprenant une partie antéro-postérieure (3), une trochlée (4), un condyle externe (5), un condyle interne (6), un plateau tibial (2) coopérant avec l'implant fémoral et un troisième condyle (11) de postéro-stabilisation, positionné entre les deux condyles externe et interne, et coopérant avec une entaille correspondante (9) ménagée dans la surface du plateau tibial, caractérisée en ce que ce troisième condyle est solidaire d'une plaque amovible (12) de fixation à la partie antéro-postérieure (3).

2. Prothèse selon la revendication 1, caractérisée en ce que la plaque (12) est fixée à la partie antéro-postérieure (3) par une colle biocompatible.

3. Prothèse selon la revendication 2, caractérisée en ce que la plaque (2) est fixée à la partie antéro-postérieure (3) par au moins un plot (14) d'ancrage fémoral traversant la plaque, et dont une extrémité (14a) est vissée dans la partie antéro-postérieure.

4. Prothèse selon la revendication 2 ou 3, caractérisée en ce que la plaque (12) portant le troisième condyle (11) est placée dans un logement (13) réalisé dans la partie antéro-postérieure (3) et dont la profondeur est sensiblement égale à l'épaisseur de la plaque (12).

5. Prothèse selon la revendication 4, caractérisée en ce que le logement (13) s'étend sur toute la largeur de la partie antéro-postérieure (3).

6. Prothèse selon la revendication 4, caractérisée en ce que la plaque (12) comporte un épaulement ou bourrelet périphérique (19) saillant de la surface de ladite plaque et de la surface de la partie antéro-postérieure (3).

7. Prothèse selon l'une des revendications 4 et 5, caractérisée en ce que le bord antérieur (13a) du logement (13) est profilé en queue d'aronde ou similaire, adapté pour recevoir un bord conjugué (12a) de la plaque (12).

8. Prothèse selon l'une des revendications 1 à 7, caractérisée en ce que l'entaille (9) du plateau tibial (2) et le condyle central (11) sont profilés de manière à constituer une butée d'arrêt de l'implant fémoral (1) en cas de flexion du genou, et la surface (7, 8, 9) de ce plateau est complémentaire des surfaces anatomiques des condyles (5, 6, 11).

9. Prothèse selon l'une des revendications 4 à 8, caractérisée en ce que le fond (24) du logement (25) est incliné vers les condyles (5,6;11) par rapport à la surface (3a) de la partie antéro-postérieure (3), et la face (26) de la plaque (27) portant le troisième condyle (11), destinée à venir en appui sur le fond du logement, est également inclinée sur la surface de la partie antéro-postérieure d'un angle (B) légèrement inférieur à celui (A) du fond du logement, ce dernier étant par exemple de 4 degrés tandis que l'inclinaison de la face d'appui de la plaque est de 3 degrés.

10. Ensemble prothétique modulable de genou, comprenant un implant fémoral (1) comportant une partie antéro-postérieure (3), une trochlée (4), un condyle externe (5), un condyle interne (6) et un troisième condyle central (11), caractérisé en ce qu'il comprend une première plaque (12) portant ledit troisième condyle central (11) de postéro-stabilisation, adaptée pour pouvoir être placée dans un logement (13) formé dans la partie antéro-postérieure (3), une seconde plaque (21) dans le bord antérieur de laquelle est réalisée une échancrure incurvée (22) dont le contour épouse celui des bords d'une échancrure (17) délimitée par les condyles externe et interne, des moyens (14, 15, 16, 23) de fixation amovible de l'une ou l'autre desdites première et seconde plaque dans le logement de la partie antéro-postérieure, et un plateau tibial (2) dans la partie centrale duquel est ménagée une entaille (9) pouvant coopérer avec le troisième condyle (11) pour assurer une postéro-stabilisation.

11. Ensemble prothétique selon la revendication 10, caractérisé en ce que lesdits moyens de fixation amovible comprennent au moins un plot (14) et de préférence deux plots (14) traversant des trous (15;23) de la plaque (12;21), et dont les extrémités (14a) peuvent être vissées dans des trous (16) du logement (13), ce ou ces plots ayant une longueur suffisante pour pouvoir assurer un ancrage osseux dans l'os spongieux du fémur, et étant munis de moyens (10) permettant leur serrage par une clé.

12. Ensemble prothétique selon la revendication 10 ou 11, caractérisé en ce que les faces externes des deux plaques (12;21) et/ou de la partie antéro-postérieure (3) sont revêtues d'une couche d'hydroxyapatite de calcium favorisant la repousse osseuse.

13. Ensemble prothétique selon la revendication 10, caractérisé en ce que le fond (24) du logement (25) est incliné vers les condyles (5,6;11) par rapport à la surface (3a) de la partie antéro-postérieure (3), et les faces (26) des plaques (27;21) destinées à venir en appui sur ce fond sont également inclinées sur la surface de la partie antéro-postérieure d'un angle (B) légèrement inférieur à celui (A) du fond du logement, ce dernier étant par exemple de 4 degrés tandis que l'inclinaison des deux faces d'appui des plaques est de 3 degrés.

## Claims

1. Total knee prosthesis, including a femoral implant (1) comprising an anteroposterior part (3), a trochlea (4), and external condyle (5), an internal condyle (6), a tibial plate (2) co-operating with the femoral implant and a third condyle (11) for posterostabilisation positioned between the two external and internal condyles and co-operating with a corresponding notch (9) provided in the surface of the tibial plate, characterised in that this third condyle is integral with a removable plate (12) for fixing to the anteroposterior part (3).

2. Prosthesis according to Claim 1, characterised in that the plate (12) is fixed to the anteroposterior part (3) by a biocompatible glue.

3. Prosthesis according to Claim 2, characterised in that the plate (2) is fixed to the anteroposterior part (3) by at least one femoral anchoring stud (14) which passes through the plate and of which one end (14a) is screwed into the anteroposterior part.

4. Prosthesis according to Claim 2 or 3, characterised in that the plate (12) bearing the third condyle (11) is placed in a recess (13) which is produced in the anteroposterior part (3) and has a depth substantially equal to the thickness of the plate (12).

5. Prosthesis according to Claim 4, characterised in that the recess (13) extends over all the width of the anteroposterior part (3).

6. Prosthesis according to Claim 4, characterised in that the plate (12) has a peripheral shoulder or bead (19) projecting from the surface of the said plate and from the surface of the anteroposterior part (3).

7. Prosthesis according to one of Claims 4 and 5, characterised in that the front edge (13a) of the recess (13) is profiled in a dovetail or similar shape, adapted so as to receive a matching edge (12a) of the plate (12).

8. Prosthesis according to one of Claims 1 to 7, characterised in that the notch (9) of the tibial plate (2) and the central condyle (11) are profiled in such a way as to constitute a stop for the femoral implant (1) in the event of flexing of the knee, and the surface (7, 8, 9) of this plate is complementary to the anatomical surfaces of the condyles (5, 6, 11).

9. Prosthesis according to one of Claims 4 to 8, characterised in that the base (24) of the recess (25) is inclined towards the condyles (5, 6; 11) with respect to the surface (3a) of the anteroposterior part (3), and the face (26) of the plate (27) bearing the third condyle (11) and intended to come to rest on the base of the recess is equally inclined on the surface of the anteroposterior part (3) by an angle (B) which is slightly less than that (A) of the base of the recess, this latter being for example 4 degrees whilst the inclination of the bearing face of the plate is 3 degrees.

10. Modulable prosthetic knee assembly comprising a femoral implant (1) having an anteroposterior part (3), a trochlea (4), an external condyle (5), an internal condyle (6) and a third central condyle (11), characterised in that it comprises a first plate (12) bearing the said third central condyle (11) for posterostabilisation, adapted so as to be able to be placed in a recess (13) formed in the anteroposterior part (3), a second plate (21) in the front edge of which is produced a curved cutout (22), the contour of which follows that of the edges of a cutout (17) delimited by the external and internal condyles, means (14, 15, 16, 23) for removable fixing of one or the other of the said first and second plates in the recess of the anteroposterior part, and a tibial plate (2) which has provided in its central part a notch (9) which can co-operate with the third condyle (11) in order to ensure posterostabilisation.

11. Prosthetic assembly according to Claim 10, characterised in that the said means for removable fixing comprise at least one stud (14) and preferably two studs (14) which pass through holes (15; 23) in the plate (12; 21), and of which the ends (14a) can be screwed into holes (16) in the recess (13), this stud or these studs being of sufficient length to be able to ensure bone anchorage in the ethmoid bone of the femur, and being provided with means (10) enabling them to be locked by a key.

12. Prosthetic assembly according to Claims 10 or 11, characterised in that the external faces of the two plates (12; 21) and/or of the anteroposterior part (3) are coated with a layer of calcium hydroxyapatite which promotes bone regrowth.

13. Prosthetic assembly according to Claim 10, characterised in that the base (24) of the recess (25) is inclined towards the condyles (5, 6; 11) with respect to the surface (3a) of the anteroposterior part (3), and the faces (26) of the plates (27; 21) intended to come to rest on this base are equally inclined on the surface of the anteroposterior part (3) by an angle (B) which is slightly less than that (A) of the base of the recess, this latter being for example 4 degrees whilst the inclination of the two bearing faces of the plates is 3 degrees.

## Patentansprüche

1. Kniegesamtprothese mit einem Oberschenkelimplantat (1), das einen Vorder-Rückabschnitt (3), eine Trochlea (4), einen äußeren Gelenkfortsatz (5), einen inneren Gelenkfortsatz (6), eine Tibiaplatte (2), die mit dem Oberschenkelimplantat zusammenwirkt, und einen dritten Gelenkfortsatz (11) zur Stabilisierung nach hinten aufweist, der zwischen dem äußeren und dem inneren Gelenkfortsatz angeordnet ist und mit einer entsprechenden, in der Oberfläche der Tibiaplatte ausgebildeten Vertiefung (9) zusammenwirkt, dadurch gekennzeichnet, daß der dritte Gelenkfortsatz fest mit einer lösbaren Platte (12) zur Befestigung an dem Vorder-Rückabschnitt (3) verbunden ist.

2. Prothese nach Anspruch 1, dadurch gekennzeichnet, daß die Platte (12) durch einen biokompatiblen Klebstoffan dem Vorder-Rückabschnitt (3) befestigt ist.

3. Prothese nach Anspruch 2, dadurch gekennzeichnet, daß die Platte (12) an dem Vorder-Rückabschnitt (3) durch wenigstens einen Stift (14) zur Oberschenkelverankerung befestigt ist, der durch die Platte hindurchgeht und dessen eines Ende (14a) in den Vorder-Rückabschnitt eingeschraubt ist.

4. Prothese nach Anspruch 2 oder 3, dadurch gekennzeichnet, daß die Platte (12), die den dritten Gelenkfortsatz (11) trägt, in einem Sitz (13) positioniert ist, welcher in dem Vorder-Rückabschnitt (3) ausgebildet ist und dessen Tiefe im wesentlichen der Dicke der Platte (12) entspricht.

5. Prothese nach Anspruch 4, dadurch gekennzeichnet, daß der Sitz (13) sich über die gesamte Breite des Vorder-Rückabschnittes (3) erstreckt.

6. Prothese nach Anspruch 4, dadurch gekennzeichnet, daß die Platte (12) einen um ihren Umfang verlaufenden Ansatz oder Wulst (19) aufweist, der von der Oberfläche der Platte und der Oberfläche des Vorder-Rückabschnittes (3) vorsteht.

7. Prothese nach einem der Ansprüche 4 und 5, dadurch gekennzeichnet, daß die Vorderkante (13a) des Sitzes (13) in Form eines Schwalbenschwanzes oder ähnlich ausgeführt ist und eine zugeordnete Kante (12a) der Platte (12) aufnehmen kann.

8. Prothese nach einem der Ansprüche 1 bis 7, dadurch gekennzeichnet, daß die Vertiefung (9) der Tibiaplatte (2) und der mittlere Gelenkfortsatz (11) so ausgeführt sind, daß sie bei Beugung des Knies einen Anschlag zur Arretierung des Oberschenkelimplantats (1) bilden, und daß die Oberfläche (7, 8, 9) dieser Platte komplementär zu den anatomischen Oberflächen der Gelenkfortsätze (5, 6, 11) ausgebildet ist.

9. Prothese nach einem der Ansprüche 4 bis 8, dadurch gekennzeichnet, daß der Boden (24) des Sitzes (25) bezüglich der Oberfläche (3a) des Vorder-Rückabschnittes (3) zu den Gelenkfortsätzen (5, 6; 11) hin geneigt ist, und daß die Fläche (26) der den dritten Gelenkfortsatz (11) tragenden Platte (27), die auf dem Boden des Sitzes zum Aufliegen kommen soll, bezogen auf die Oberfläche des Vorder-Rückabschnittes mit einem Winkel (B) geneigt ist, der etwas kleiner ist als der Winkel (A) des Bodens des Sitzes, wobei letzterer Winkel beispielsweise 4 Grad beträgt, während die Neigung der Auflagefläche der Platte 3 Grad beträgt.

10. Modularer Knieprothesenbausatz mit einem Oberschenkelimplantat (1), welches einen Vorder-Rückabschnitt (3), eine Trochlea (4), einen äußeren Gelenkfortsatz (5), einen inneren Gelenkfortsatz (6) und einen dritten, mittleren Gelenkfortsatz (11) aufweist, dadurch gekennzeichnet, daß er
- eine erste Platte (12), welche den dritten, mittleren Gelenkfortsatz (11) zur Stabilisierung nach hinten trägt und in einem in dem Vorder-Rückabschnitt (3) ausgebildeten Sitz (13) angeordnet werden kann,
- eine zweite Platte (21), in deren Vorderkante eine gekrümmte Ausnehmung (22) ausgebildet ist, deren Umriß dem der Kanten einer Ausnehmung (17) entspricht, die von dem äußeren und dem inneren Gelenkfortsatz begrenzt wird,
- Einrichtungen (14, 15, 16, 23) zur lösbaren Befestigung entweder der ersten oder der zweiten Platte in dem Sitz des Vorder-Rückabschnittes, und
- eine Tibiaplatte (2) aufweist, in deren Mittelabschnitt eine Vertiefung (9) ausgebildet ist, die mit dem dritten Gelenkfortsatz (11) zusammenwirken kann, um eine Stabilisierung nach hinten zu gewährleisten.

11. Prothesenbausatz nach Anspruch 10, dadurch gekennzeichnet, daß die Einrichtungen zur lösbaren Befestigung wenigstens einen Stift (14) und vorzugsweise zwei Stifte (14) umfassen, die durch Löcher (15; 23) der Platte (12; 21) hindurchgehen und deren Enden (14a) in Löcher (16) des Sitzes (13) geschraubt sein können, wobei dieser Stift bzw. diese Stifte eine ausreichende Länge hat bzw. haben, um eine Knochenverankerung in der Spongiosa des Oberschenkelknochens zu gewährleisten, und mit Einrichtungen (10) versehen ist bzw. sind, die sein bzw. ihr Festziehen mit Hilfe eines Schlüssels ermöglichen.

12. Prothesenbausatz nach Anspruch 10 oder 11, dadurch gekennzeichnet, daß die Außenflächen der beiden Platten (12; 21) und/oder des Vorder-Rückabschnittes (3) mit einer Schicht aus Kalzium-Hydroxyapatit überzogen sind, die das Nachwachsen des Knochens fördert.

13. Prothesenbausatz nach Anspruch 10, dadurch gekennzeichnet, daß der Boden (24) des Sitzes (25) bezüglich der Oberfläche (3a) des Vorder-Ruckabschnittes (3) zu den Gelenkfortsätzen (5, 6; 11) hin geneigt ist, und daß die Flächen (26) der Platten (27;21), die auf diesem Boden zum Aufliegen kommen sollen, bezogen auf die Oberfläche des Vorder-Rückabschnittes mit einem Winkel (B) geneigt sind, der etwas kleiner ist als der Winkel (A) des Bodens des Sitzes, wobei letzterer Winkel beispielsweise 4 Grad beträgt, während die Neigung der beiden Auflageflächen der Platten 3 Grad beträgt.
